# EUROPEAN PATENT APPLICATION

(11) **EP 2 548 891 A1**
(43) Date of publication of application: **23.01.2013**
(21) Application number: 11174361.3
(22) Date of filing: 18.07.2011
(51) Int. Cl.: C07K 16/28, C07K 16/30

(54) **Human monoclonal antibody against the gamma subunit of the acetylcholine receptor for use in the treatment of rhabdomyosarcoma.**

(71) Applicant: Universiteit Maastricht, 6211 LK Maastricht (NL); Academisch Ziekenhuis Maastricht, 6229 HX Maastricht (NL)
(72) Inventor: Martinez-Martinez, Maria Pilar, 6213 JL Maastricht (NL); Vrolix, Kathleen, 3900 Overpelt (BE); Molenaar, Pieter Cornelis, 4130 Esneux (BE); De Baets, Marc Hubert Valentijn, 3630 Maasmechelen (BE); Losen, Mario Rene, 6213 JL Maastricht (NL)
(74) Representative: Habets, Winand

(57) **Abstract**

The disclosure is in the field of biological sciences, more in particular immunology. The disclosure provides a human monoclonal antibody directed against the gamma subunit of the acetylcholine receptor. This subunit is almost exclusively expressed in fetal tissue and in several neoplasms among which rhabdomyosarcomas. More in particular, the disclosure provides an antibody and a method for using this antibody in the treatment of rhabdomyosarcomas.

## Description

### Field of the invention

The invention is in the field of biological sciences, more in particular immunology. The invention provides a human monoclonal antibody directed against the gamma subunit of the muscle type acetylcholine receptor. This subunit is almost exclusively expressed in fetal muscle tissue and in several neoplasms among which rhabdomyosarcomas.

### Background of the invention

Rhabdomyosarcomas (RMS), according to the WHO classification, comprise three types of tumors sharing skeletal muscle differentiation while showing distinct genetic and biological features: 1) embryonal with a characteristic loss of heterozygocity at 11 p15; 2) the more aggressive alveolar, most of which show pathognomonic Pax3 or Pax7-FKHR fusion genes due to the translocations t(2;13)(q35;q14) and t(1;13)(p36;q14), respectively; and 3) the rare pleomorphic RMS with complex genetic aberrations and preferential occurrence in adults.

As a group, RMS are the most frequent soft tissue tumors in children and have a poor prognosis. In particular, metastatic alveolar RMS in children more than 10 years old are often refractory to all current therapies including adjuvant bone marrow transplantation, resulting in 5-year survival rates of 5-20%. Immunotherapies or other (e.g. genetic) specific targeting strategies have not yet been applied due to the absence of a well defined RMS-specific gene target and the lack of suitable immunotherapeutic tools. However, it was shown previously that strong expression of fetal acetylcholine receptor (AChR) mRNA and protein is virtually pathognomonic for non-innervated rhabdomyoblasts and rhabdomyotubes and, thus, for the vast majority of human embryonal and alveolar RMS; by contrast, fetal AChR expression is virtually absent in other tissues and in tumors without a rhabdomyoblast component.

The nicotinic AChR of skeletal muscle is a pentameric ion channel composed of five subunits. The fetal isoform is composed of 2[alpha], 1 [beta], 1 [gamma] and 1 [delta]-subunits, while the adult isoform exhibits an [epsilon]-subunit instead of the [gamma]-subunit. During the later stages of development in uterus, the fetal AChR is gradually replaced by the adult isoform in virtually all innervated muscles. After birth, strong expression of fetal AChR is restricted to a few muscle fibres of unknown function occurring in extraoccular muscles and myoid cells of the thymus that are physiologically non-innervated muscle cells. However, high-level re-expression of fetal AChR can occur after birth following denervation, including denervating neuromuscular diseases.

In adult human innervated skeletal muscles, therefore, fetal AChR expression is exceedingly low. Only traces of [gamma]-subunit mRNA can be detected by RT-PCR and RNAse protection assay, and very little by Northern blot. In addition, it has been shown that the mothers of newborns with arthrogryposis multiplex caused by the placental transfer of maternal antibodies specific to fetal AchR can be completely asymptomatic, suggesting that there is minimal expression of AChR [gamma]-subunit protein on normal adult muscle.

It has recently been shown that immunotoxins coupled to a specific antibody for the gamma subunit of AChR are effective in the treatment of RMS (Gattenlöhner et al., J. Biomed Biotechnol. 2010, Art ID 187621).

So far, antibodies against the gamma subunit of AChR have mostly been isolated from mice and rats, however for the treatment of human diseases, a human antibody is preferred in order to avoid unwanted immunologic cross-reactions. Gattenlöhner et al (J. Biomed Biotechnol. 2010, Art ID 187621) describe the use of a human recombinant autoantibody isolated from a combinatorial cDNA thymus library from a patient with thymic lymphoid hyperplasia (Matthews et al., Laboratory investigation, 82, 1407-1417 (2002)). Whereas this antibody coupled to a Pseudomonas exotoxin A (ETA) inhibited rhabdomyosarcoma growth in vitro and in a murine transplantation model, the antibody itself was not effective against the tumor.

High affinity human monoclonal antibodies against the gamma subunit of AChR are desired.

### Summary of the invention

We isolated human monoclonal antibodies from the thymus of a patient suffering from myasthenia gravis. One of these antibodies (clone P90-131) appeared to be specific for the gamma subunit of the acetylcholine receptor (AChR). A second monoclonal antibody from this the same patient (clone P90-65) also bound to the anti-AChR. We characterized the P90-131 antibody by sequencing its complimentarity determining regions (CDRs).

The invention therefore relates to a specific binding member reactive with the gamma subunit of the muscle type acetylcholine receptor, comprising at least one CDR selected from the group consisting of HCDR 1 (SEQ ID NO: 11), HCDR2 (SEQ ID NO: 12), HCDR3 (SEQ ID NO: 13), LCDR1 (SEQ ID NO: 14), LCDR2 (SEQ ID NO: 15) and LCDR3 (SEQ ID NO: 16).

### Detailed description of the invention

We set out to isolate a human monoclonal antibody that is specific for the gamma subunit of AChR. We therefore relied on the natural immune repertoire of a patient with myasthenia gravis.

We used an improved Epstein-Barr virus (EBV) immortalization technique on human thymic B cells [1, 2]. In order to study the complete B cell repertoire of the thymus, we characterized the antibodies that are produced by monoclonal immortalized B cells. We focused on acetylcholine receptor (AChR)-antibody positive myasthenia gravis (MG) (i.e. AChR-MG) because thymic B cells have been implicated in its etiology as explained below. We isolated a full-size human monoclonal antibody directed against the gamma subunit of the AChR, which exerted pathogenic effects on the AChR in vitro. So far, the production of human monoclonal (auto-) antibodies has been proven to be difficult and only a small number of human monoclonal anti-AChR antibodies have been cloned from MG patients. The majority of anti-AChR antibodies have been isolated using phage display libraries [3-8]. However, the antibodies produced by phage display do not entirely reflect the natural human immune response because the original pairing of heavy and light chains is lost. Production of hybridomas with human B cells or EBV transformation overcomes this drawback.

Myasthenia gravis (MG) is an auto-immune disease that is characterized by skeletal muscle weakness [9, 10]. In the majority of MG patients, auto-antibodies bind to the acetylcholine receptor (AChR) at the postsynaptic membrane of the NMJ [11]. Anti-AChR antibodies can impair neuromuscular transmission by four mechanisms, antigenic modulation of the AChR [12, 13], complement-mediated lysis of the postsynaptic membrane [14], loss of AChR associated proteins such as rapsyn, voltage gated sodium channels and utrophin, and, less frequently, direct inhibition of AChRs [15, 16]. Whereas these pathogenic effects of the AChR-antibodies are well understood, the original trigger for auto-antibody production itself remains unknown.

To date, only few groups have isolated human monoclonal anti-AChR antibodies from MG patients. One B cell line reactive with human AChR has been generated from a hyperplastic thymus using immortalization with Epstein Barr virus (EBV) [17]. This method showed a low efficiency and produced mainly IgM antibodies. Applying the hybridoma technology, IgM anti-Torpedo AChR monoclonal antibodies have been isolated from PBMC's [18, 19]. Since these antibodies were not able to immunoprecipitate human AChR in the standard assay, one continued investigating other methods to isolate human anti-AChR antibodies. Combinatorial phage display libraries have been constructed from both thymic lymphocytes [3-6] and peripheral blood lymphocytes [7, 8], and their screening resulted in the successful isolation of anti-AChR specific Fabs. However, the disadvantage of this technique is that it randomly pairs heavy and light chains, thereby not revealing information on their original pairing. IgM anti-AChR monoclonal antibodies have also been isolated from transgenic humanized mice [20].

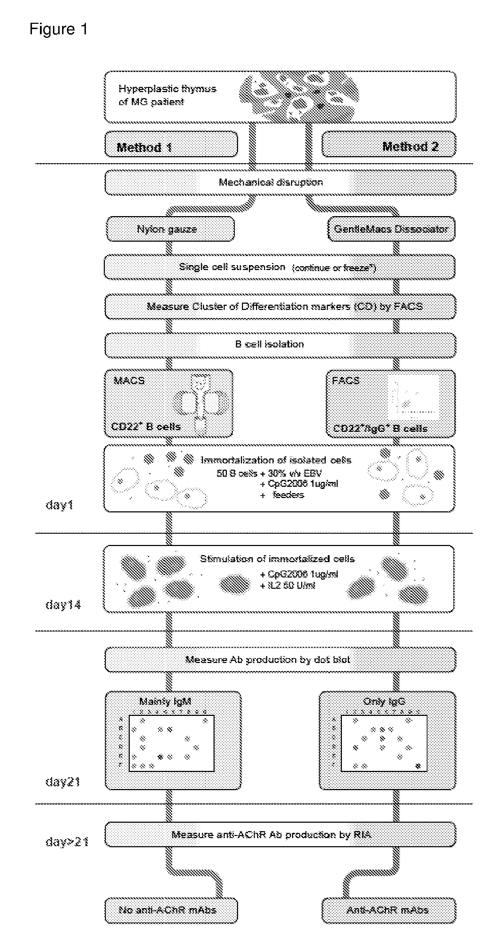

Recently, a more efficient B cell immortalization procedure has been published [1], which we further improved for the characterization of autoimmune B cells [2]. This enabled us to study the autoantibody repertoire from thymic B cells from AChR-MG patients. Using Method 1 (Figure 1), a large number of anti-striated muscle reactive B cell clones were isolated from the MG thymi, while Method 2, which is selective for IgG producing B cells, resulted in the isolation of 2 anti-AChR reactive monoclonal cell lines from 1 out of 2 MG patients.

For the first time, we now successfully isolated human IgG monoclonal antibody against the gamma subunit of AChR, from the thymus of MG patients using EBV immortalization.

These results also emphasize the usefulness of our method to study the auto-antibody repertoire in MG patients and to identify antibodies in other less characterized auto-immune diseases.

Also, the antibody now allows the specific targeting of cells carrying the gamma subunit of AChR, such as cells from rhabdomyosarcomas.

We determined the specific binding sites of these monoclonal antibodies by sequencing their complementarity determining regions.

Complementarity determining regions (CDRs) are regions within antibodies or T cell receptors where these proteins complement an antigen's shape. Thus, CDRs determine the protein's affinity and specificity for specific antigens. The CDRs are the most variable part of the molecule, and contribute to the diversity of these molecules, allowing the antibody and the T cell receptor to recognize a vast repertoire of antigens.

In the amino acid sequence of a variable domain of an antigen receptor there are three CDRs (CDR1, CDR2 and CDR3), arranged non-consecutively. Since the antigen receptors are typically composed of two variable domains (on two different polypeptide chains, heavy and light chain), there are six CDRs for each antigen receptor that can collectively come into contact with the antigen. A typical single antibody molecule has two antigen receptors, wherefore it contains twelve CDRs.

Within the variable domain, CDR1 and CDR2 are found in the variable (V) region of a polypeptide chain, and CDR3 includes some of V, all of diverse (D, heavy chains only) and joint (J), and some of the constant (C) regions. CDR3 is the most variable region.

Since most sequence variation associated with immunoglobulins and T cell receptors are found in the CDRs, these regions are sometimes referred to as hypervariable regions.[1] Among these, CDR3 shows the greatest variability as it is encoded by a recombination of the VJ in the case of a light chain region and VDJ in the case of heavy chain regions.

To clone human monoclonal anti-AChR antibodies from the thymus of AChR-MG patients, we used two different methods to isolate and immortalize thymic B cells (Fig. 1). Before the immortalization, we determined the phenotypic characteristics of B cells from the MG thymus by FACS. Results are shown in Table 1. We found that the percentage of CD22+ cells (i.e. mature B cells) in total thymocytes highly varied between MG patients, ranging from 8 to 36%. The percentage of CD22+IgG+ thymocytes ranged from 1.6 to 4.5%, indicating that the majority of CD22+ thymic B cells express other immunoglobulin isotypes. The majority of CD22+ B cells, ranging from 68-95%, were found to express the EBV receptor CD21, thereby suggesting that they would be susceptible to EBV immortalization.

CD22+ thymic B cells from 3 MG patients (P21, P28 and P42) were immortalized following isolation by magnetic cell sorting (Fig. 1, Method 1). Immortalization efficiencies, based on the amount of clones that actively grew until sufficient amounts of cells could be frozen, ranged from 10 to 29% (Table 1). The antibody production of growing B cell clones was measured by dot blot and more than 60% was found to produce IgM antibodies. We then selectively enriched CD22+IgG+ B cells using FACS sorting (Method 2) before immortalization. These CD22+IgG+ B cells were isolated from the thymus of 2 other MG patients (P82 and P90). All B cell clones immortalized by Method 2 were positive for IgG production. Finally, we determined the monoclonality rate in the immortalized B cell lines by spectratyping analysis. We obtained about 90% monoclonal and 10% biclonal B cell lines (data not shown), thereby eliminating the need for further subcloning.

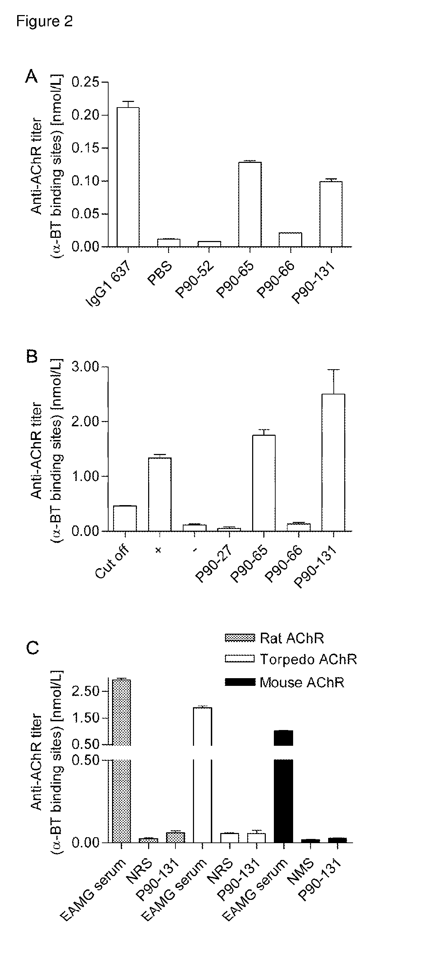

The reactivity of immortalized B cell lines against human AChR was tested using RIAs. From the B cell clones immortalized by Method 1, none produced antibodies with specific AChR reactivity. Conversely, Method 2 yielded two clones with reactivity against the human AChR. As shown in Figure 2, the AChR binding activity of P90-65 and P90-131 was confirmed by two different RIAs, using human AChR either from the TE 671 cell line (Fig. 2A) or from leg muscles (Fig. 2B).

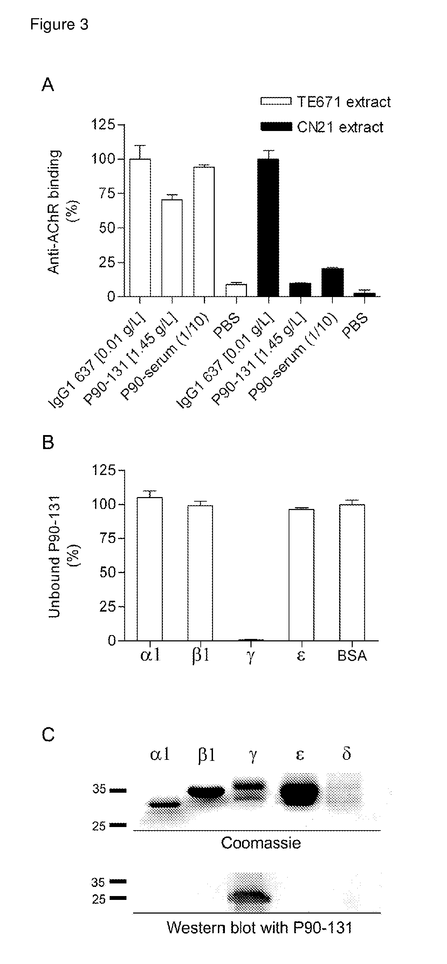

We studied the specificity of P90-131 against AChR from different species, but no reactivity against rat, mouse or Torpedo AChR was detected (Fig. 2C). In addition, P90-131 did not bind to AChRs on the NMJ of monkey muscle tissue in immunostainings (data not shown). Then, we investigated whether P90-131 recognized adult or fetal AChR, by using AChR extracted from the CN 21 or TE 671 cell line, respectively. P90-131 as well as the serum of patient P90 showed reactivity against the fetal AChR and P90-131 did not bind to the adult AChR (Fig. 3A). The immunoabsorption experiment (Fig. 3B) and Western blot (Fig. 3C) clearly showed binding of P90-131 to the gamma-AChR subunit only, thereby confirming its specific reactivity against the fetal AChR (alpha2,beta, gamma, delta).

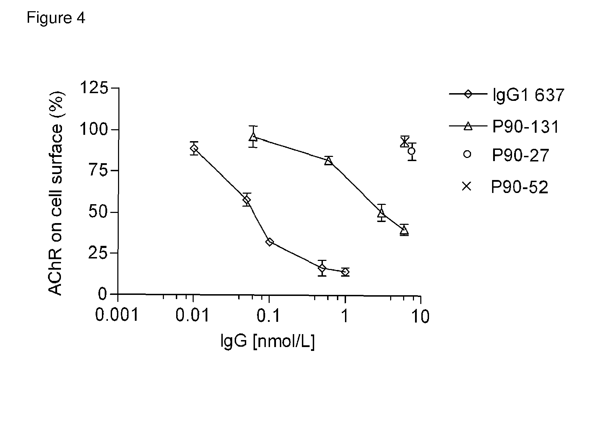

Because the internalization of cell surface AChR is an important mechanism by which anti-AChR antibodies exert their pathogenic effect in vivo [12, 13, 21], we tested the modulating effect of P90-131 on cultured TE 671 cells. As shown in Figure 4, P90-131 effectively reduced surface AChR levels in a concentration-dependent manner, reaching a maximum reduction of 60% when a concentration of ~6 nM of IgG was used. Human mAb IgG1 637, which is directed against the human AChR alpha subunit and known to efficiently reduce surface AChR was used as a positive control [22]. As expected, two IgG-producing B cell clones, negative for anti-AChR antibodies in the RIA, did not cause significant reduction in surface AChR levels. These results clearly show that P90-131 exerts pathogenic effects in vitro.

In addition, the isotyping assay determined that clone P90-131 produced IgG1 antibodies (data not shown). This means that P90-131 might activate the classical pathway of the complement system, thereby implying that P90-131 could also exert pathogenic effects in vivo, by activating the membrane attack complex of complement.

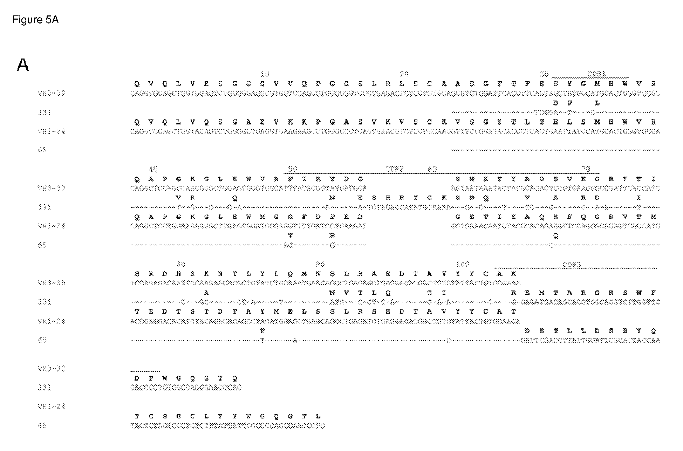

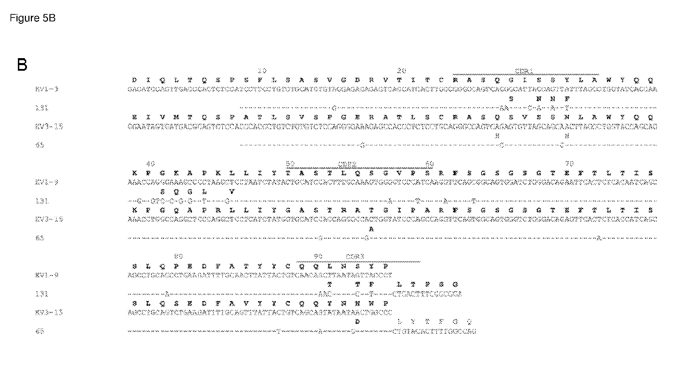

Sequence analysis of anti-AChR positive clones P90-65 and P90-131 revealed that their VH gene fragments had most likely differentiated from the germ-line genes V1-24*01 (M99642) and V3-30*02 (L26401) to which they showed 96.8% and 74.7% of homology, respectively (Fig. 5A). The high rate of mutations in mutational hot spots of P90-131 (data not shown) suggests that these B cells have passed through a germinal center to undergo affinity maturation towards the antigen. In addition, the ratios of replacement to silent mutations (R/S) observed in the complementarity determining regions (CDR) were higher than in the framework regions (FR) in both clones, indicating antigen-driven selection. Comparison of IgVH nucleotide sequences of P90-65 with P90-131 did not show significant similarity. The VL sequences of P90-65 and P90-131 showed 96.5% and 89.1 % homology, respectively, to their germ-line genes V3-15*01 (M23090) and V1-9*01 (K02096) (Fig. 5B). The IgVL nucleotide sequences of P90-65 and P90-131 display almost 75% of homology (data not shown). When comparing the VH and VL gene usage of P90-65 and P90-131 with previously published human anti-AChR antibodies no preferential usage of certain IgV genes was observed. Interestingly, the VH and DH gene family used in clone P90-131 matched with those used by anti-AChR Fabs that were published by Fostieri and colleagues [4].

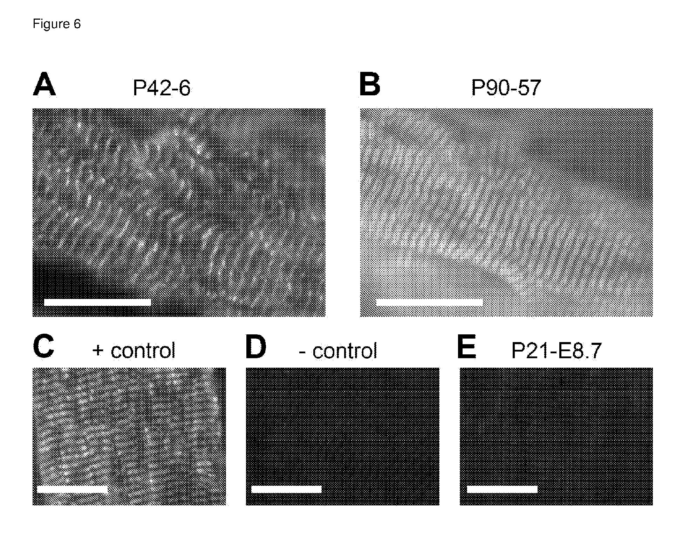

In addition to anti-AChR antibodies, MG patients often present with antibodies against striational muscle proteins [23]. Therefore, we screened all immortalized B cells for striated muscle reactivity. Representative results of the immunohistochemical stainings are shown in Figure 6. Approximately 25% of CD22+ B cell clones, immortalized by Method 1, showed reactivity against striational proteins (P21: 7/19; P28: 5/22; P42: 6/38) both from the IgM (13 out of 18; 72%) and IgG (5 out of 18; 28%) subtype. Of the CD22+IgG+ B cell clones immortalized by Method 2, only one monoclonal line against striational muscle proteins was identified (P82: 0/20; P90:1/30; 2%). Different staining patterns that were observed suggested specificity for different striational antigens. Therefore, ELISAs were performed to identify antibodies against actin, myosin, alpha-actinin and titin, but none of the striational antibodies showed specific reactivity against any of these proteins (data not shown). We also tested the anti-striated muscle reactivity of immortalized B cells isolated from PBMC's of multiple sclerosis (MS) patients and healthy individuals, but no striational antibodies were detected

In conclusion, P90-131 is the first anti-gamma AChR antibody to be described to exert pathogenic effects when gamma-subunit containing AChRs are present by inducing AChR internalization in vitro. Recently, the fetal AChR (alpha2, beta,gamma,delta) has also been described as a specific marker of human rhabsomyosarcoma (RMS), and RMS cells have been shown to be destroyed by an immunotoxin-labeled human anti-gamma AChR Fab fragment.. This indicates that our full-size anti-gamma AChR antibody, because of its strong binding and modulating effects, could potentially be used to refine this treatment strategy in RMS patients.

The isolation of representative striational and anti-AChR antibodies from the thymus of MG patients led to the conclusion that our improved method of B cell immortalization is a valuable tool to characterize the B cell repertoire in the autoimmune thymus. An adaptation of our method to isolate specific antibodies at high throughput may even lead to the development of new antibodies with therapeutic characteristics.

The present invention provides specific binding members for the gamma subunit of the muscle type acetylcholine receptor (GMTACR), in particular human and/or primate GMTACR and/or murine GMTACR. Preferred embodiments within the present invention are antibody molecules, whether whole antibody (e.g. IgG, such as IgG4) or antibody fragments (e.g. scFv, Fab, dAb). Antibody antigen binding regions are provided, as are antibody VH and VL domains. Within VH and VL domains are provided complementarity determining regions, CDR's, which may be provided within different framework regions, FR's, to form VH or VL domains as the case may be. An antigen binding site may consist of an antibody VH domain and/or a VL domain.

An antigen binding site may be provided by means of arrangement of CDR's on non-antibody protein scaffolds such as fibronectin or cytochrome B etc. [31, 32]. Scaffolds for engineering novel binding sites in proteins have been reviewed in detail by Nygren et al [32]. Protein scaffolds for antibody mimics are disclosed in WO/0034784 in which the inventors describe proteins (antibody mimics) which include a fibronectin type III domain having at least one randomised loop. A suitable scaffold into which to graft one or more CDR's, such as for example a set of HCDR's, may be provided by any domain member of the immunoglobulin gene superfamily.

In one aspect, the present invention provides a specific binding member for GMTACR, such as a binding member specific for the gamma subunit of GMTACR, comprising at least one CDR selected from the group consisting of HCDR 1 (SEQ ID NO: 11), HCDR2 (SEQ ID NO: 12), HCDR3 (SEQ ID NO: 13), LCDR1 (SEQ ID NO: 14), LCDR2 (SEQ ID NO: 15) and LCDR3 (SEQ ID NO: 16). In a preferred embodiment, the invention relates to a specific binding member for human GMTACR.

In another aspect, the invention relates to a specific binding member for GMTACR comprising an antibody antigen-binding site which is composed of a antibody VH domain and a antibody VL domain and which comprises a set of CDR's, wherein the VH domain comprises HCDR 1 (SEQ ID NO: 11), HCDR2 (SEQ ID NO: 12) and HCDR3 (SEQ ID NO: 13) and the VL domain comprises LCDR1 (SEQ ID NO: 14), LCDR2 (SEQ ID NO: 15) and LCDR3 (SEQ ID NO: 16). In a further preferred embodiment, the invention relates to a specific binding member for GMTACR comprising an antibody antigen-binding site which is composed of a human antibody VH domain and a human antibody VL domain as described above.

Further provided by the present invention is a binding member comprising a VH domain comprising a set of CDR's comprising HCDR1, HCDR2 and HCDR3.

Further provided by the present invention is a binding member comprising a VL domain comprising a set of CDR's comprising LCDR1, LCDR2 and LCDR3.

A specific binding member comprising an antibody antigen-binding domain comprising such a VH and a VL domain is also provided by the present invention.

An antibody antigen-binding site composed of a VH domain and a VL domain is formed by six loops of polypeptide: three from the light chain variable domain (VL) and three from the heavy chain variable domain (VH). Analysis of antibodies of known atomic structure has elucidated relationships between the sequence and three-dimensional structure of antibody combining sites [26,27]. These relationships imply that, except for the third region (loop) in VH domains, binding site loops have one of a small number of main-chain conformations: canonical structures. The canonical structure formed in a particular loop has been shown to be determined by its size and the presence of certain residues at key sites in both the loop and in framework regions [26,27].

This study of sequence-structure relationship can be used for prediction of those residues in an antibody of known sequence, but of an unknown three-dimensional structure, which are important in maintaining the three-dimensional structure of its CDR loops and hence maintain binding specificity. These predictions can be backed up by comparison of the predictions to the output from lead optimization experiments.

In accordance with further aspects of the present invention there is provided a specific binding member which competes for binding to the GMTACR antigen with any specific binding member which both binds the GMTACR antigen and comprises a specific binding member, HCDR, LCDR, VH and/or VL domain disclosed herein, or HCDR3 disclosed herein, or variant of any of these. Competition between binding members may be assayed easily in vitro, for example using ELISA and/or by tagging a specific reporter molecule to one binding member which can be detected in the presence of other untagged binding member(s), to enable identification of specific binding members which bind the same epitope or an overlapping epitope.

Thus, a further aspect of the present invention provides a specific binding member comprising a human antibody antigen-binding site which competes for binding to GMTACR with a binding member comprising at least one of the CDRs disclosed herein, in particular with the human monoclonal antibody P90-131.

As used herein, the term specific binding member describes a member of a pair of molecules which have binding specificity for one another. The members of a specific binding pair may be naturally derived or wholly or partially synthetically produced. One member of the pair of molecules has an area on its surface, or a cavity, which specifically binds to and is therefore complementary to a particular spatial and polar organisation of the other member of the pair of molecules. Thus the members of the pair have the property of binding specifically to each other. Examples of types of specific binding pairs are antigen-antibody, antigen-T-cell receptor, biotin-avidin, hormone-hormone receptor, receptor-ligand, enzyme-substrate. The present invention is mainly concerned with antigen-antibody or antigen-T-cell receptor type interactions.

The term "antibody molecule" describes an immunoglobulin whether natural or partly or wholly synthetically produced. The term also covers any polypeptide or protein comprising an antibody binding domain. Antibody fragments which comprise an antigen binding domain are molecules such as Fab, scFv, Fv, dAb, Fd; and diabodies.

It is possible to take monoclonal and other antibodies and use techniques of recombinant DNA technology to produce other antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. See, for instance, EP-A-184187 , GB 2188638A or EP-A-239400 , and a large body of subsequent literature. A hybridoma or other cell producing an antibody may be subject to genetic mutation or other changes, which may or may not alter the binding specificity of antibodies produced.

As antibodies can be modified in a number of ways, the term "antibody molecule" should be construed as covering any specific binding member or substance having an antibody antigen-binding domain with the required specificity. Thus, this term covers antibody fragments and derivatives, including any polypeptide comprising an immunoglobulin binding domain, whether natural or wholly or partially synthetic. Chimeric molecules comprising an immunoglobulin binding domain, or equivalent, fused to another polypeptide are therefore included. Cloning and expression of chimeric antibodies are described in EP-A-0120694 and EP-A-0125023 , and a large body of subsequent literature.

Further techniques available in the art of antibody engineering have made it possible to isolate human and humanised antibodies. For example, human hybridomas can be made as described by Kontermann et al [29]. Phage display, another established technique for generating specific binding members has been described in detail in many publications such as Kontermann et al [29] and WO92/01047 (discussed further below). Transgenic mice in which the mouse antibody genes are inactivated and functionally replaced with human antibody genes while leaving intact other components of the mouse immune system, can be used for isolating human antibodies to human antigens [40].

Synthetic antibody molecules may be created by expression from genes generated by means of oligonucleotides synthesized and assembled within suitable expression vectors, for example as described by Knappik et al. J. Mol. Biol. (2000) 296, 57-86 or Krebs et al. Journal of Immunological Methods 254 2001 67-84. It has been shown that fragments of a whole antibody can perform the function of binding antigens. Examples of binding fragments are (i) the Fab fragment consisting of VL, VH, CL and CH1 domains; (ii) the Fd fragment consisting of the VH and CH1 domains; (iii) the Fv fragment consisting of the VL and VH domains of a single antibody; (iv) the dAb fragment (Ward, E.S. et al., Nature 341, 544-546 (1989), McCafferty et al (1990) Nature, 348, 552-554) which consists of a VH domain; (v) isolated CDR regions; (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al, Science, 242, 423-426, 1988 ; Huston et al, PNAS USA, 85, 5879-5883, 1988); (viii) bispecific single chain Fv dimers (PCT/US92/09965) and (ix) "diabodies", multivalent or multispecific fragments constructed by gene fusion (WO94/13804 ; P. Holliger et al, Proc. Natl. Acad. Sci. USA 90 6444-6448, 1993). Fv, scFv or diabody molecules may be stabilised by the incorporation of disulphide bridges linking the VH and VL domains (Y. Reiter et al, Nature Biotech, 14, 1239-1245, 1996). Minibodies comprising a scFv joined to a CH3 domain may also be made (S. Hu et al, Cancer Res., 56, 3055-3061, 1996).

Where bispecific antibodies are to be used, these may be conventional bispecific antibodies, which can be manufactured in a variety of ways (Holliger, P. and Winter G. Current Opinion Biotechnol. 4, 446-449 (1993)), e.g. prepared chemically or from hybrid hybridomas, or may be any of the bispecific antibody fragments mentioned above. Examples of bispecific antibodies include those of the BiTE technology in which the binding domains of two antibodies with different specificity can be used and directly linked via short flexible peptides. This combines two antibodies on a short single polypeptide chain. Diabodies and scFv can be constructed without an Fc region, using only variable domains, potentially reducing the effects of anti-idiotypic reaction.

Bispecific diabodies, as opposed to bispecific whole antibodies, may also be particularly useful because they can be readily constructed and expressed in E.coli. Diabodies (and many other polypeptides such as antibody fragments) of appropriate binding specificities can be readily selected using phage display (WO94/13804) from libraries. If one arm of the diabody is to be kept constant, for instance, with a specificity directed against GMTACR, then a library can be made where the other arm is varied and an antibody of appropriate specificity selected. Bispecific whole antibodies may be made by knobs-into-holes engineering (J. B. B. Ridgeway et al, Protein Eng., 9, 616-621, 1996).

The term "antigen-binding domain" describes the part of an antibody molecule which comprises the area which specifically binds to and is complementary to part or all of an antigen. Where an antigen is large, an antibody may only bind to a particular part of the antigen, which part is termed an epitope. An antigen binding domain may be provided by one or more antibody variable domains (e.g. a so-called Fd antibody fragment consisting of a VH domain). Preferably, an antigen binding domain comprises an antibody light chain variable region (VL) and an antibody heavy chain variable region (VH).

The term "specific" is used to refer to the situation in which one member of a specific binding pair will not show any significant binding to molecules other than its specific binding partner(s). The term is also applicable where e.g. an antigen binding domain is specific for a particular epitope which is carried by a number of antigens, in which case the specific binding member carrying the antigen binding domain will be able to bind to the various antigens carrying the epitope.

The term "comprise" is generally used herein in the sense of include, that is to say permitting the presence of one or more other and/or further and/or additional features or components. The term "comprise" encompasses the term "consisting of".

The term "isolated" refers to the state in which specific binding members of the invention, or nucleic acid encoding such binding members, will generally be in accordance with the present invention. Isolated members and isolated nucleic acid will be free or substantially free of material with which they are naturally associated such as other polypeptides or nucleic acids with which they are found in their natural environment, or the environment in which they are prepared (e.g. cell culture) when such preparation is by recombinant DNA technology practiced in vitro or in vivo. Members and nucleic acid may be formulated with diluents or adjuvants and still for practical purposes be isolated - for example the members will normally be mixed with gelatin or other carriers if used to coat microtitre plates for use in immunoassays, or will be mixed with pharmaceutically acceptable carriers or diluents when used in diagnosis or therapy. Specific binding members may be glycosylated, either naturally or by systems of heterologous eukaryotic cells (e.g. CHO or NS0 (ECACC 85110503) cells, or they may be (for example if produced by expression in a prokaryotic cell) unglycosylated.

Recombinant proteins expressed in prokaryotic bacterial expression systems are not glycosylated while those expressed in eukaryotic systems such as mammalian or insect cells are glycosylated. Proteins expressed in insect cells however differ in glycosylation from proteins expressed in mammalian cells.

By "substantially as set out" it is meant that the relevant CDR or VH or VL domain of the invention will be either identical or highly similar to the specified regions of which the sequence is set out herein. By "highly similar" it is contemplated that from 1 to 5, preferably from 1 to 4 such as 1 to 3 or 1 or 2, or 3 or 4, amino acid substitutions may be made in the CDR and/or VH or VL domain. Such highly similar CDRs are also part of the present invention.

The structure for carrying a CDR or a set of CDR's of the invention will generally be of an antibody heavy or light chain sequence or substantial portion thereof in which the CDR or set of CDR's is located at a location corresponding to the CDR or set of CDR's of naturally occurring VH and VL antibody variable domains encoded by rearranged immunoglobulin genes. The structures and locations of immunoglobulin variable domains may be determined by reference to Kabat, E.A. et al, Sequences of Proteins of Immunological Interest. 4th Edition. US Department of Health and Human Services. 1987, and updates thereof, now available on the Internet at (http://immuno.bme.nwu.edu or find "Kabat" using any search engine). [28]

CDR's can also be carried by other scaffolds such as fibronectin or cytochrome B [31, 32].

Preferably, a CDR amino acid sequence substantially as set out herein is carried as a CDR in a human variable domain or a substantial portion thereof. The HCDR3 sequences substantially as set out herein represent preferred embodiments of the present invention and it is preferred that each of these is carried as a HCDR3 in a human heavy chain variable domain or a substantial portion thereof.

Variable domains employed in the invention may be obtained from any germ-line or rearranged human variable domain, or may be a synthetic variable domain based on consensus sequences of known human variable domains. A CDR sequence of the invention (e.g. CDR3) may be introduced into a repertoire of variable domains lacking a CDR (e.g. CDR3), using recombinant DNA technology.

For example, Marks et al (Bio/Technology, 1992, 10:779-783) describe methods of producing repertoires of antibody variable domains in which consensus primers directed at or adjacent to the 5' end of the variable domain area are used in conjunction with consensus primers to the third framework region of human VH genes to provide a repertoire of VH variable domains lacking a CDR3. Marks et al further describe how this repertoire may be combined with a CDR3 of a particular antibody. Using analogous techniques, the CDR3-derived sequences of the present invention may be shuffled with repertoires of VH or VL domains lacking a CDR3, and the shuffled complete VH or VL domains combined with a cognate VL or VH domain to provide specific binding members of the invention. The repertoire may then be displayed in a suitable host system such as the phage display system of WO92/01047 or any of a subsequent large body of literature, including Kay, B.K., Winter, J., and McCafferty, J. (1996) Phage Display of Peptides and Proteins: A Laboratory Manual, San Diego: Academic Press , so that suitable specific binding members may be selected. A repertoire may consist of from anything from 10<4> individual members upwards, for example from 10<6> to 10<8> or 10<10> members. Other suitable host systems include yeast display, bacterial display, T7 display, ribosome display and so on. For a review of ribosome display for see Lowe D and Jermutus L, 2004, Curr. Pharm, Biotech, 517-27 , also WO92/01047 .

Analogous shuffling or combinatorial techniques are also disclosed by Stemmer (Nature, 1994, 370:389-391), who describes the technique in relation to a [beta]-lactamase gene but observes that the approach may be used for the generation of antibodies.

A further alternative is to generate novel VH or VL regions carrying CDR-derived sequences of the invention using random mutagenesis of one or more selected VH and/or VL genes to generate mutations within the entire variable domain. Such a technique is described by Gram et al (1992, Proc. Natl. Acad. Sci., USA, 89:3576-3580), who used error-prone PCR. In preferred embodiments one or two amino acid substitutions are made within a set of HCDR's and/or LCDR's.

Another method which may be used is to direct mutagenesis to CDR regions of VH or VL genes. Such techniques are disclosed by Barbas et al, (1994, Proc. Natl. Acad. Sci., USA, 91:3809-3813) and Schier et al (1996, J. Nol. Biol. 263:551-567).

All the above described techniques are known as such in the art and in themselves do not form part of the present invention. The skilled person will be able to use such techniques to provide specific binding members of the invention using routine methodology in the art.

In accordance with the present invention, compositions provided may be administered to individuals. Administration is preferably in a "therapeutically effective amount", this being sufficient to show benefit to a patient. Such benefit may be at least amelioration of at least one symptom. The actual amount administered, and rate and time-course of administration, will depend on the nature and severity of what is being treated. Prescription of treatment, e.g. decisions on dosage etc, is within the responsibility of general practitioners and other medical doctors. Appropriate doses of antibody are well known in the art; see Ledermann J.A. et al. (1991) Int. J. Cancer 47: 659-664 ; Bagshawe K.D. et al. (1991) Antibody, Immunoconjugates and Radiopharmaceuticals 4: 915-922 .

The precise dose will depend upon a number of factors, including whether the antibody is for diagnosis or for treatment, the size and location of the area to be treated, the precise nature of the antibody (e.g. whole antibody, fragment or diabody), and the nature of any detectable label or other molecule attached to the antibody. A typical antibody dose will be in the range 100 microgram to 1 gram for systemic applications, and 1 microgram to 1 milligram for topical applications. Typically, the antibody will be a whole antibody. This is a dose for a single treatment of an adult patient, which may be proportionally adjusted for children and infants, and also adjusted for other antibody formats in proportion to molecular weight. Treatments may be repeated at daily, twice-weekly, weekly or monthly intervals, at the discretion of the physician. In preferred embodiments of the present invention, treatment is periodic, and the period between administrations is about two weeks or more, preferably about three weeks or more, more preferably about four weeks or more, or about once a month.

Specific binding members of the present invention will usually be administered in the form of a pharmaceutical composition, which may comprise at least one component in addition to the specific binding member.

Thus pharmaceutical compositions according to the present invention, and for use in accordance with the present invention, may comprise, in addition to active ingredient, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. intravenous.

Pharmaceutical compositions for oral administration may be in tablet, capsule, powder or liquid form. A tablet may comprise a solid carrier such as gelatin or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

A composition may be administered alone or in combination with other treatments, either simultaneously or sequentially dependent upon the condition to be treated.

Specific binding members of the present invention may be formulated in liquid or solid forms depending on the physicochemical properties of the molecule and the route of delivery. Formulations may include excipients, or combinations of excipients, for example: sugars, amino acids and surfactants. Liquid formulations may include a wide range of antibody concentrations and pH. Solid formulations may be produced by lyophilisation, spray drying, or drying by supercritical fluid technology, for example. Formulations of anti-GMTACR will depend upon the intended route of delivery: for example, formulations for pulmonary delivery may consist of particles with physical properties that ensure penetration into the deep lung upon inhalation; topical formulations may include viscosity modifying agents, which prolong the time that the drug is resident at the site of action.

The present invention provides a method comprising causing or allowing binding of a specific binding member as provided herein to GMTACR. As noted, such binding may take place in vivo, e.g. following administration of a specific binding member, or nucleic acid encoding a specific binding member, or it may take place in vitro, for example in ELISA, Western blotting, immunocytochemistry, immuno-precipitation, affinity chromatography, or cell based assays.

The present invention further provides an isolated nucleic acid encoding a specific binding member of the present invention. Nucleic acid may include DNA and/or RNA. In a preferred aspect, the present invention provides a nucleic acid which codes for a CDR or set of CDR's or VH domain or VL domain or antibody antigen-binding site or antibody molecule, e.g. scFv or IgG4, of the invention as defined above.

The present invention also provides constructs in the form of plasmids, vectors, transcription or expression cassettes which comprise at least one polynucleotide as above.

The present invention also provides a recombinant host cell which comprises one or more constructs as above. A nucleic acid encoding any CDR or set of CDR's or VH domain or VL domain or antibody antigen-binding site or antibody molecule, e.g. scFv or IgG4 as provided, itself forms an aspect of the present invention, as does a method of production of the encoded product, which method comprises expression from encoding nucleic acid therefore. Expression may conveniently be achieved by culturing under appropriate conditions recombinant host cells containing the nucleic acid. Following production by expression a VH or VL domain, or specific binding member may be isolated and/or purified using any suitable technique, then used as appropriate.

Specific binding members, VH and/or VL domains, and encoding nucleic acid molecules and vectors according to the present invention may be provided isolated and/or purified, e.g. from their natural environment, in substantially pure or homogeneous form, or, in the case of nucleic acid, free or substantially free of nucleic acid or genes origin other than the sequence encoding a polypeptide with the required function. Nucleic acid according to the present invention may comprise DNA or RNA and may be wholly or partially synthetic. Reference to a nucleotide sequence as set out herein encompasses a DNA molecule with the specified sequence, and encompasses a RNA molecule with the specified sequence in which U is substituted for T, unless context requires otherwise.

Systems for cloning and expression of a polypeptide in a variety of different host cells are well known. Suitable host cells include bacteria, mammalian cells, plant cells, yeast and baculovirus systems and transgenic plants and animals. Mammalian cell lines available in the art for expression of a heterologous polypeptide include Chinese hamster ovary (CHO) cells, HeLa cells, baby hamster kidney cells, NS0 mouse melanoma cells, YB2/0 rat myeloma cells, human embryonic kidney cells, human embryonic retina cells and many others. A common, preferred bacterial host is E. coli.

The expression of antibodies and antibody fragments in prokaryotic cells such as E. coli is well established in the art. For a review, see for example Plockthun, A. Bio/Technology 9: 545-551 (1991). Expression in eukaryotic cells in culture is also available to those skilled in the art as an option for production of a specific binding memberfor example Chadd HE and Chamow SM (2001) 110 Current Opinion in Biotechnology 12: 188-194 , Andersen DC and Krummen L (2002) Current Opinion in Biotechnology 13: 117 , Larrick JW and Thomas DW (2001) Current opinion in Biotechnology 12:411-418.

Suitable vectors can be chosen or constructed, containing appropriate regulatory sequences, including promoter sequences, terminator sequences, polyadenylation sequences, enhancer sequences, marker genes and other sequences as appropriate. Vectors may be plasmids, viral e.g. 'phage, or phagemid, as appropriate. For further details see, for example, Molecular Cloning: a Laboratory Manual: 3rd edition, Sambrook and Russell, 2001, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Second Edition, Ausubel et al. eds., John Wiley & Sons, 1988 , Short Protocols in Molecular Biology: A Compendium of Methods from Current Protocols in Molecular Biology, Ausubel et al. eds., John Wiley & Sons, 4th edition 1999 . The disclosures of Sambrook et al. and Ausubel et al. (both) are incorporated herein by reference.

Thus, a further aspect of the present invention provides a host cell containing nucleic acid as disclosed herein. Such a host cell may be in vitro and may be in culture. Such a host cell may be in vivo. In vivo presence of the host cell may allow intracellular expression of the specific binding members of the present invention as "intrabodies" or intracellular antibodies. Intrabodies may be used for gene therapy [30].

Therewith, the invention preferably relates to a binding member such as an isolated antibody comprising a heavy chain and a light chain, each comprising three Complementarity Determining Regions (CDRs), characterized in that the heavy chain comprises the amino acid sequence DFGLHW (SEQ ID NO: 11) in CDR1, FIRNDESRRYGKSDQYYVDAVRD (SEQ ID NO: 12) in CDR2 and AREMTARGRSWFDP (SEQ ID NO: 13) in CDR3 and that the light chain comprises the amino acid sequence RASQSINNFLA (SEQ ID NO: 14) in CDR1, TASTLQSGVPS (SEQ ID NO: 15) in CDR2 and QQTNTFPLT (SEQ ID NO: 16) in CDR3.

In a preferred embodiment, the antibody according to the inventions is of human origin.

It is particularly preferred when the antibody as described above also contains the sequences according to SEQ ID NO: 9 and SEQ ID NO: 10.

Now that the variable regions, in particular the CDRs of the antibodies of the invention have been disclosed herein, one skilled in the art may now produce recombinant antibodies, single chain antibodies and other types of specific binding members such as antibodies using the information as contained herein.

The invention therefore also relates to a recombinant antibody comprising at least one of the CDRs described above.

The antibody may be coupled to a cytotoxic agent in order to improve its toxic effect on cells in vitro and in vivo. The invention therefore also relates to an antibody as described above attached to a cytotoxic agent.

The nucleic acid molecules as disclosed herein may be useful in constructing recombinant vectors for the expression of binding molecules and recombinant antibodies. The invention therefore relates to a nucleic acid molecule with a nucleotide sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7 and SEQ ID NO: 8.

The invention also relates to a vector comprising a nucleic acid sequence according to claim 7. Such a vector may conveniently be expressed in a prokaryotic or eukaryotic vector. The invention therefore relates to a cell comprising the vector according to the invention..

Tumors often express fetal antigens. Tumors expressing the fetal AChR comprising the gamma subunit, may effectively be targeted with the antibody according to the invention. The invention therefore relates to an antibody, a nucleic acid or a vector as described above for use in the treatment of a tumor.

Rhabdomyosarcomas are characterized by the expression of fetal AChR. The invention is therefore in particular directed towards an antibody for use as described above wherein the tumor is a rhabdomyosarcoma.

In addition, the compounds and compositions of the invention may be used to decorate cells in vitro. The invention is therefore also directed towards a method for the in vitro labeling of tumor cells expressing a fetal acetylcholine receptor wherein said tumor cell is contacted with an antibody, a nucleic acid or a vector according to the invention.

### Legend to the figures

Figure 1. Schematic overview of thymic B cell immortalizations.

Two different methods were used to immortalize B cells. The hyperplastic thymus of MG patients was mechanically disrupted using either nylon gauze (Method 1, left panel) or the GentleMacs dissociator (Method 2, right panel). Single cell suspensions were used fresh or were frozen until later time points (*). Then, the expression of different B cell CD markers in total thymocytes was measured using FACS. CD22+ or CD22+IgG+ B cells were isolated from total thymocytes by magnetic sorting or fluorescent sorting, respectively. Isolated B cells were simultaneously stimulated with CpG2006 and immortalized with 30% (v/v) EBV-containing supernatant. Following two weeks, EBV-containing supernatant was removed and B cells were stimulated with CpG2006 and IL-2 during 7 days. Immortalization efficiency was verified on day 21 by microscopic examination of cell growth and by measuring antibody production by dot blot. Immortalizing CD22+ thymic B cells mainly yielded IgM antibodies, while immortalizing CD22+IgG+ B cells only produced IgG antibodies. At later time points, the antibodies were screened for anti-AChR reactivity using RIA. Method 2 successfully resulted in the isolation of anti-AChR antibodies from the thymus of AChR-MG patients.

Figure 2. Immortalized B cell clones produce anti-human AChR antibodies.
(A) Specificity of immortalized B cell clones against human AChR was tested by RIA using AChR isolated from the TE 671 cell line. mAb IgG1 637 [0.01 g/L] was used as a positive control for AChR binding. Non-concentrated culture supernatant of the B cell lines was used with a total Ig concentration of approximately 1 µg/mL. Values represent the average of at least in triplo measurements.
(B) Specificity of immortalized B cell clones against human AChR was tested using the IBL diagnostic kit. The first bar represents the cut-off value, as provided with the kit. Anti-AChR human serum (+) and normal human serum (-) were used as positive and negative controls, respectively. Values represent the average of in duplo measurements.
(C) Specificity of P90-131 against non-human AChR was tested using AChR isolated from rat or mouse denervated muscle tissue or from the electric organ of Torpedo californica. Anti-AChR antibody positive rat serum (EAMG serum) and normal rat or mouse serum (NRS/NMS) were used as positive and negative controls, respectively, for binding against rat, mouse and Torpedo AChR. Values represent the average of in duplo measurements.

Figure 3. P90-131 specifically binds to the fetal AChR.
(A) Specificity of P90-131 (concentrated culture supernatants) against fetal (α2βγδ) and adult (α2βεδ) AChR was tested using AChR isolated from TE 671 and CN 21 cell line, respectively. IgG1 637 was used as a positive control for both cell lines. The results are shown as percentage compared to 0.01 g/L IgG1 637. Values represent the average of at least in duplo measurements.
(B) Specificity of P90-131 (concentrated culture supernatants) was tested against extracellular domains of human AChR subunits using immunoabsorption. The results are shown as percentage compared to the BSA control. Values represent the average of in triplo measurements.
(C) Specificity of P90-131 (concentrated culture supernatants) was tested against extracellular domains of human AChR subunits using Western blot. AChR subunits alpha, beta, gamma, epsilon and delta are visualized by Coomassie staining (upper panel). Incubation with culture supernatants of P90-131 specifically shows reactivity of P90-131 against the gamma-subunit of the AChR only (lower panel).

Figure 4. P90-131 modulates surface AChR on TE 671 cells.

Confluent TE 671 cells were incubated with cycloheximide and culture supernatants of immortalized B cell clones. The percentage of AChR on cell surface is calculated as described in the materials and methods section. mAb IgG1 637 was used as a positive control for AChR internalization. Non-concentrated culture supernatant was used for all immortalized B cell lines. Values represent the average of in triplicate measurements from three independent experiments.

Figure 5. Sequence alignment of anti-AChR antibodies with germline sequences. Nucleotide sequence alignment of the VH and VL regions of clone 131 and 65 are shown together with the closest matching germline counterparts. Amino acids are shown in bold. Dashes represent nucleotide identity with the germline sequence. The CDR regions are indicated.
(A) Heavy chain alignments. Clone 131 and 65 were closest related to V3-30 (L26401) and V1-24 (M99642) germline sequences, respectively.
(B) Light chain alignments. K light chains of clone 131 and 65 resembled KV1-9 (K02096) and KV3-15 (M23090) germline sequences, respectively.

Figure 6. Immortalized B cell clones produces striational antibodies.

Monkey muscle tissue was stained by immunofluorescence with culture supernatants (non-concentrated) of immortalized B cell clones. (A) Positive clone P42-6, (B) Positive clone P90-57, (C) Positive control provided with the testing system (ScimedX), (D) Negative control provided with the testing system (Scimedx), (E) Negative clone P21-E8.7. Scale bar represents 20 µm.

### Examples

### Patient information

MG patients underwent thymectomy at the Academic Hospital of Maastricht in the Netherlands. Informed consent was obtained from all patients and the study was approved by the local medical ethical committee (NL22271.068.08). Clinical data of MG patients are summarized in Table 1.

**Table 1. Clinical characteristics of AChR-MG patients and phenotype of their thymic B cells**

| Patient | P21 | P28 | P42 | P82 | P90 |
|---|---|---|---|---|---|
| Sex | female | female | female | female | female |
| Disease severity (MGFA) | IIIa | I | IVb | IIIb | IVb |
| Age at thymectomy (y) | 29 | 41 | 24 | 28 | 23 |
| Thymic histology | FH | FH | FH | FH | FH |
| Anti-AChR Ab titer (nM) | 104 | 90 | 24 | >20 | >500 |
| Age at onset (years) | 26 | 38 | 23 | 28 | 23 |
| MG symptoms | Generalized | Ocular/bulbar | Generalized | Generalized | Generalized |
| Treatment | Mestinon | Mestinon | Mestinon | Mestinon | Mestinon |
| Comorbidities | RA | / | Diabetis | Scoliosis | / |
| CD22⁺ thymocytes | 36.61% | 11.0% | 8.13% | ND | ND |
| CD22⁺IgG⁺ thymocytes | 4.15% | 4.45% | 3.43% | 1.9%* | 1.6%* |
| CD21⁺ of CD22⁺ thymocytes | 95.19% | 78.56 % | 67.77% | ND | ND |
| Immortalization method | 1 | 1 | 1 | 2 | 2 |
| Immortalization efficiency^{§} | 10.5% | 29% | 12.5% | 7.5% | 25% |
| Number of growth-positive clones | 63 | 84 | 182 | 54 | 187 |
| IgG⁺ immortalized clones | 16% | 40% | 19% | 100 % | 100 % |
| Monoclonality rate | 84% | 94% | 93% | 90% | 83% |
| Anti-AChR reactivity | 0 | 0 | 0 | 0 | 2 |

| | | | | | |
|---|---|---|---|---|---|
| FH: follicular hyperplasia; RA: rheumatoid arthritis; ND: not determined. Disease severity is scored according to the Myasthenia Gravis Foundation of America (MGFA) clinical classification. * Values were measured with FACSAria instead of the FACSCalibur system. ^{§}Immortalization efficiency represents the percentage of clones that became immortalized and could be kept in culture long enough to reach an estimated total number of 30 million cells. | | | | | |

### Example 1: preparation of monoclonal antibody.

Thymus tissue obtained after thymectomy was mechanically disrupted by rubbing it through a nylon gauze or by using the gentIeMACS Tissue Dissociator (Miltenyi Biotec, Bergisch Gladbach, Germany) according to the manufacturer's instructions. The crude cell suspension was collected in RPMI medium and passed through a 30 micrometer cell strainer to remove large cell clumps and fat tissue. Single cell suspensions were used fresh (P90) or were stored in liquid N2 until experiments started (P21, P28, P42 and P82).

CD22+ B cells were isolated from the total thymocyte cell population by binding to CD22 microbeads (Miltenyi Biotec) and separating them on a magnetic column (P21, P28 and P42). Alternatively, CD22+IgG+ B cells were isolated by staining total thymocytes with anti-CD22 and anti-IgG antibodies and subsequently enriching positively labeled cells using the FACSAria II cell sorter (all from BD Biosciences, San Jose, CA, USA) (P82 and P90). B cell immortalization was performed as described previously [2]. In short, selected B cells were seeded in 96 U-bottom microwell plates in RPMI medium containing 10% fetal bovine serum (Bodinco, Alkmaar, The Netherlands), 1 mM sodium pyruvate (Gibco Invitrogen, Carlsbad, USA), 50 U/mL penicillin, 50 µg/mL streptomycin, 1 microgram CpG 2006 (Invivogen, San Diego, USA) and 50 U/mL interleukin-2 (IL-2) (Roche, Basel, Switzerland), in the presence of EBV (supernatant of the B95-8 cell line; 30% v/v). As feeder cells, 105 irradiated (83 Gy) PBMCs from healthy donors were used. After 2 weeks, EBV was removed and cells were restimulated with 1 microgram CpG 2006 and 50 U/ml IL-2 for another week, after which the immortalization was verified by microscopic examination of cell growth and by screening the culture supernatants for antibody production. Growth-positive and antibody-producing clones were kept in culture until they grew confluently in a 75 cm2 flask and could be frozen in N2. Figure 1 depicts the different procedures which were used to immortalize thymic B cells.

Dot blotting was used to measure the antibody production of immortalized B cell lines. Culture supernatants (5 microliter) of all B cell lines were spotted on a nitrocellulose membrane (Bio-Rad Laboratories, Hercules, USA), and allowed to dry for 15 minutes. After blocking the membrane with 5% non-fat dry milk in phosphate buffered saline (PBS) for 30 minutes at room temperature (RT), it was incubated for one hour with rabbit anti-human IgG labeled with horseradish peroxidase (HRP) (1/100 in blocking buffer; Dako, Glostrup, Denmark) or rabbit anti-human IgM HRP (1/50 in blocking buffer; Dako). Following 5 washing steps with 0.05% Triton X-100 in PBS, the membrane was incubated with substrate solution containing di-aminobenzidine (DAB, 1 mg/mL), Tris-HCl (50 mmol/L) and 0.03% H2O2 until spots were visible. Human IgG (IgG1 637, [22]) or IgM (Sigma Aldrich, St-Louis, USA) (ranging from 500 µg/mL to 0.5 µg/mL) was included as a positive control, and culture medium was used as a negative control.

To determine the IgG isotype of B cell clone P90-131, the PeliClass human IgG subclass Plus kit (Sanquin, Amsterdam, The Netherlands) was used according to the manufacturer's instructions.

Radioimmunoassays (RIA) were performed to measure anti-human AChR antibodies in culture supernatants of immortalized B cell clones. Culture supernatant was incubated with fetal AChR from the TE 671 human rhabdomyosarcoma cell line, 125Iodine-labeled α-bungarotoxin (1/250; PerkinElmer, Waltham, USA) and normal human serum overnight at 4 degrees Celsius as described previously [22]. For detection of antibodies binding to the ε-ACHR subunit, the CN 21 cell line was used. The CN 21 has been produced by D. Beeson and colleagues by stable transfection of TE 671 cell with a plasmid driving the production of the AChR epsilon subunit, resulting in the predominant expression of adult AChR [24]. To determine the cross-reactivity of anti-AChR antibodies between different species, rat and mouse denervated muscle tissue and the electric organ of Torpedo californica were used as source of AChR. Anti-AChR antibodies that bound the radioactively-labeled receptor in the antibody complex were measured with the Wizard2 automatic gamma counter (PerkinElmer).

Alternatively, anti-AChR antibodies in the culture supernatants of immortalized B cell clones were measured against AChR isolated from human amputated legs by using the commercially available AChR antibody kit (IBL, Hamburg, Germany) according to the manufacturer's instructions.

Immunoabsorption of mAb by sepharose-immobilized ECDs and RIA

Extracellular domains (ECDs) of the human AChR subunits α1, β1, γ and ε were expressed as soluble proteins in P. pastoris [25]. After purification by nickel-affinity chromatography, they were individually immobilized on CNBr activated sepharose (GE Healthcare, Fairfield, CT, USA) to a ratio of 0.1 mg protein / 0.1 g sepharose. A control column with immobilized bovine serum albumin (BSA) was also prepared. 10 µL of the culture supernatant of P90-131 was incubated with 1 µg of each of the sepharose-immobilized ECDs, or sepharose-BSA (in a total volume of 160 microliter PBS, pH 7.4), for 2 h at 4°C. Then the samples were briefly centrifuged to pellet the sepharose. Unbound anti-AChR antibodies in the supernatant (duplicate samples of 40 microliter each) were assayed by RIA (RSR, Cardiff, UK) according to the manufacturer's protocol. The antibodies detected after incubation with sepharose-BSA were taken as the 100% value. The immunoabsorptions were performed in three independent experiments.

The ECDs (3-6 µg) of the α1, β1, γ and subunits were resolved by SDS-PAGE and then transferred onto a nitrocellulose membrane. The blot was blocked with 5% non-fat dry milk in PBS and then probed with the culture supernatant of P90-131 for 16 h at 4°C, followed by HRP-labeled rabbit anti-human IgG (1:1000 in PBS; Dako, Glostrup, Denmark). The molecular masses of proteins were estimated relatively to the electrophoretic mobility of cotransferred prestained molecular mass markers (Fermentas, USA). To visualize the α1, β1, γ and ε subunits on the gel, a coomassie staining was performed.

### Example 2: Antigenic modulation of AChR

Confluently grown TE 671 cells were incubated with culture supernatants of B cell lines, culture medium (negative control) or with monoclonal anti-AChR antibody IgG1 637 (10 nM, positive control) [22] for three hours at 37°C. The synthesis of new proteins was prevented by adding cycloheximide (40 microMolar). After washing with prewarmed PBS, the cells were incubated for one hour at 37°C with 125I-labeled α-bungarotoxin (1/5000 in culture medium; PerkinElmer) to label the remaining AChR on the cell surface. Following three washing steps, cells were lysed with 0.5 M sodium hydroxide and the amount of labeled AChR was measured using the Wizard2 automatic gamma counter (PerkinElmer). The non-specific binding of 1251-labeled α-bungarotoxin to TE 671 cells was measured in samples that had been pre-incubated with unlabeled α-bungarotoxin. The amount of AChR on the cells was calculated by subtracting the non-specific radioactivity from each measurement (Δcpm). The maximum amount of cell surface 1251- α-bungarotoxin labeled AChR (Δcpm max) is the cell-bound radioactivity on confluent cells where plain culture medium is maintained during the steps before labeling. The percentage of surface AChR was calculated as follows: % AChR on cell surface= (Δcpm in the presence of Ig/ Δcpm max) * 100.

The anti-striated monkey (macaques) muscle antibody immunofluorescence test system (ScimedX, Denville, USA) was used to detect striational antibodies in the culture supernatant of immortalized B cells. Stainings were performed according to the manufacturer's instructions. To detect antibodies that bind proteins of the NMJ, 30 µm cryosections from intercostal monkey muscles were used instead. Sections were cut using the Leica CM3050 cryostate (Leica Microsystems GmbH, Wetzlar, Germany), dried, fixed in acetone at 4 degrees Celsius for 10 minutes and dried again for 30 minutes. Subsequently, the sections were incubated with culture supernatants for three hours at RT. To confirm the localization of AChR in the rhesus muscle end-plates, sections were double stained with Alexa 594-conjugated α-bungarotoxin (1/300 in PBS; Molecular Probes, Leiden, The Netherlands) for 30 minutes at RT. Then, they were incubated with sheep-anti-human IgG FITC (1/100 in PBS; The Binding Site, Birmingham, UK) for 60 minutes at RT. After washing with PBS, coverslips were mounted with 30% glycerol in PBS.

### Example 3: Spectratyping and sequencing analysis

RNA was isolated from the immortalized B cells by using the high pure RNA isolation kit (Roche) according to the manufacturer's instructions. The isolated RNA was subsequently converted into cDNA with the Reverse transcription system kit (Promega) according to the manufacturer's instructions. To analyze the variable region of the B cell receptor heavy and light chain locus, a PCR was performed with the following reagents: PCR buffer (100 mM Tris-HCl, 500 mM KCI, 15 mM MgC12 and 0.1% gelatine), 0.25 mM dNTPs (GE Healthcare), 10 pmol FR1, FR2, and FR3 forward primer mix (Sigma Aldrich), 10 pmol Primer Biomed JH-con FAM (Sigma Aldrich), one unit Jumpstart Red Taq polymerase (Sigma Aldrich) and 100 ng cDNA, using the PTC200 DNA Engine Thermal Cycler PCR (Scientific Support, Hayward, USA). The primers used to amplify VH genes have been described previously [2]. In the case of VK genes, the primers used in 5' were as follows: VK1: GAC ATC CAG ATG ACC CAG TCT CC; VK2: GAT GTT GTG ATG ACT CAG TCT CC; VK3: GAA ATT GTG TTG ACG CAG TCT CC; VK4: GAC ATC GTG ATG ACC CAG TCT CC; VK5: GAA ACG ACA CTC ACG CAG TCT CC; VK6: GAA ATT GTG CTG ACT CAG TCT CC. In 3', the consensus JK primer CTT ACG TTT GAT CTC CAG CTT GGT CCC was used. PCR products were analyzed by electrophoresis on a 2% agarose gel and photographed by the Molecular Imager Gel Doc XR System (Bio-Rad Laboratories). To measure the length of different frameworks, the PCR-product was added to an internal size standard and formamide and analyzed by the genetic analyzer AB13100 (Applied Biosystems, Foster City, USA) which detects the FAM-labels of the PCR-products in order to identify monoclonal or polyclonal B cell clones. This spectratyping procedure can detect clonal populations with a sensitivity of 0.5-5%. To sequence the heavy and light chain of the antibody, the PCR-product was used as template for the sequence reaction, based on single strand primer extension by Taq-polymerase in a PCR-reaction. The mix for the sequence reaction contained PCR product (10% v/v), JH or JK con primer (3.2 pmol/µL) (Sigma Aldrich), Big Dye v1.1 (10% v/v) (Applied Biosystems) and sequence buffer (15% v/v). The PCR-product was amplified in a PE 9700 thermocycler (PerkinElmer) and analyzed by the genetic analyzer AB13100 (Applied Biosystems). The IgVH and IgVL regions were subsequently sequenced in the forward direction by means of a VH and VK family specific forward primer using the same procedure. Forward and reverse sequences were aligned using the BLAST alignment tool (bI2seq, NCBI) in order to establish the complete V(D)J region sequence. The obtained sequences were compared to the BLAST immunoglobulin database (NCBI) and found novel.

### References

1. Traggiai, E., et al., An efficient method to make human monoclonal antibodies from memory B cells: potent neutralization of SARS coronavirus. Nat Med, 2004. 10(8): p. 871-5.
2. Fraussen, J., et al., A novel method for making human monoclonal antibodies. J Autoimmun, 2010. 35(2): p. 130-4.
3. Farrar, J., et al., Diverse Fab specific for acetylcholine receptor epitopes from a myasthenia gravis thymus combinatorial library. Int Immunol, 1997. 9(9): p. 1311-8.
4. Fostieri, E., et al., Isolation of potent human Fab fragments against a novel highly immunogenic region on human muscle acetylcholine receptor which protect the receptor from myasthenic autoantibodies. Eur J Immunol, 2005. 35(2): p. 632-43.
5. Graus, Y.F., et al., Human anti-nicotinic acetylcholine receptor recombinant Fab fragments isolated from thymus-derived phage display libraries from myasthenia gravis patients reflect predominant specificities in serum and block the action of pathogenic serum antibodies. J Immunol, 1997. 158(4): p. 1919-29.
6. Matthews, I., et al., Antibodies to acetylcholine receptor in parous women with myasthenia: evidence for immunization by fetal antigen. Lab Invest, 2002. 82(10): p. 1407-17.
7. Rey, E., et al., Characterization of human anti-acetylcholine receptor monoclonal autoantibodies from the peripheral blood of a myasthenia gravis patient using combinatorial libraries. Clin Immunol, 2000. 96(3): p. 269-79.
8. Zeidel, M., et al., Genetic and functional characterization of human autoantibodies using combinatorial phage display libraries. Ann N Y Acad Sci, 1995. 764: p. 559-64.
9. Conti-Fine, B.M., M. Milani, and H.J. Kaminski, Myasthenia gravis: past, present, and future. J Clin Invest, 2006. 116(111): p. 2843-54.
10. Vincent, A., D. Beeson, and B. Lang, Molecular targets for autoimmune and genetic disorders of neuromuscular transmission. Eur J Biochem, 2000. 267(23): p. 6717-28.
11. Lindstrom, J.M., et al., Antibody to acetylcholine receptor in myasthenia gravis. Prevalence, clinical correlates, and diagnostic value. Neurology, 1976. 26(l 1): p. 1054-9.
12. Drachman, D.B., et al., Myasthenic antibodies cross-link acetylcholine receptors to accelerate degradation. N Engl J Med, 1978. 298(20): p. 1116-22.
13. Heinemann, S., et al., Modulation of acetylcholine receptor by antibody against the receptor. Proc Natl Acad Sci U S A, 1977. 74(7): p. 3090-4.
14. Chamberlain-Banoub, J., et al., Complement membrane attack is required for endplate damage and clinical disease in passive experimental myasthenia gravis in Lexis rats. Clin Exp Immunol, 2006. 146(2): p. 278-86.
15. Almon, R.R., C.G. Andrew, and S.H. Appel, Serum globulin in myasthenia gravis: inhibition of alpha-bungarotoxin binding to acetylcholine receptors. Science, 1974. 186(4158): p. 55-7.
16. Lang, B., et al., Plasma from myasthenia gravis patients reduces acetylcholine receptor agonist-induced Na+ flux into TE671 cell line. J Neuroimmunol, 1988. 19(1-2): p. 141-8.
17. Kamo, I., et al., Monoclonal antibody to acetylcholine receptor cell line established from thymus of patient with Myasthenia gravis. Science, 1982. 215(4535): p. 995-7.
18. Blair, D.A., et al., Monoclonal antibodies to acetylcholine receptor secreted by human x human hybridomas derived from lymphocytes of a patient with myasthenia gravis. Immunol Invest, 1986. 15(4): p. 351-64.
19. Cardona, A., et al., Human IgG monoclonal autoantibodies against muscle acetylcholine receptor. direct evidence for clonal heterogeneity of the antiself humoral response in myasthenia gravis. J Neuroimmunol, 1994. 53(l): p. 9-16.
20. Protopapadakis, E., et al., Isolation and characterization of human anti-acetylcholine receptor monoclonal antibodies from transgenic mice expressing human immunoglobulin loci. Eur J Immunol, 2005. 35(6): p. 1960-8.
21. Losen, M., et al., Increased expression of rapsyn in muscles prevents acetylcholine receptor loss in experimental autoimmune myasthenia gravis. Brain, 2005. 128(Pt 10): p. 2327-37.
22. van der Neut Kolfschoten, M., et al., Anti-inflammatory activity of human IgG4 antibodies by dynamic Fab arm exchange. Science, 2007. 317(5844): p. 1554-7.
23. Vrolix, K., et al., The auto-antigen repertoire in myasthenia gravis. Autoimmunity, 2010. 43(5-6): p. 380-400.
24. Beeson, D., et al., A transfected human muscle cell line expressing the adult subtype of the human muscle acetylcholine receptor for diagnostic assays in myasthenia gravis. Neurology, 1996. 47(6): p. 1552-5.
25. Kostelidou, K., et al., Expression and characterization of soluble forms of the extracellular domains of the beta, gamma and epsilon subunits of the human muscle acetylcholine receptor. FEBS J, 2006. 273(15): p. 3557-68.
26. Chothia C. et al. Journal Molecular Biology (1992) 227, 799-817 .
27. Al-Lazikani, et al. Journal Molecular Biology (1997) 273(4), 927-948 .
28. Kabat E A et al (1991): Sequences of Proteins of Immunological Interest, 5th Edition. US Department of Health and Human Services, Public Service, NIH, Washingt on.
29. Kontermann R and Dubel Stefan; (2001) Antibody Engineering, Springer Laboratory Manuals
30. Marasco WA (1997) Gene Therapy, 4(1): 11
31. Koide et al (1998). Journal of Molecular Biology, Vol 284:1141-1151 .
32. Nygren et al (1997). Current Opinion in Structural Biology, Vol 7:463-469

## Claims

1. A specific binding member reactive with the gamma subunit of the muscle type acetylcholine receptor, comprising at least one CDR selected from the group consisting of HCDR 1 (SEQ ID NO: 11), HCDR2 (SEQ ID NO: 12), HCDR3 (SEQ ID NO: 13), LCDR1 (SEQ ID NO: 14), LCDR2 (SEQ ID NO: 15) and LCDR3 (SEQ ID NO: 16).

2. A specific binding member according to claim 1 which is an isolated antibody

3. An isolated antibody according to claim 2 comprising a heavy chain and a light chain, each comprising three Complementarity Determining Regions (CDRs), **characterized in that** the heavy chain comprises the amino acid sequence DFGLHW in CDR1, FIRNDESRRYGKSDQYYVDAVRD in CDR2(SEQ ID NO: 12 and AREMTARGRSWFDP in CDR3(SEQ ID NO: 13) and that the light chain comprises the amino acid sequence RASQSINNFLA in CDR1 (SEQ ID NO: 14), TASTLQSGVPS in CDR2 (SEQ ID NO: 15) and QQTNTFPLT in CDR3(SEQ ID NO: 16)..

4. An isolated antibody according to claims 2 or 3 of human origin.

5. An isolated antibody according to claims 2 - 4 comprising the amino acid sequences according to SEQ ID NO: 9 and SEQ ID NO: 10.

6. A specific binding member according to claims 1 - 5 which is a recombinant antibody

7. A specific binding member according to claims 1 - 5 attached to a cytotoxic agent.

8. Nucleic acid encoding a specific binding member or antibody according to claims 1 - 7.

9. Nucleic acid molecule according to claim 8 comprising a nucleotide sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7 and SEQ ID NO: 8.

10. Vector comprising a nucleic acid sequence according to claims 8 or 9.

11. A cell comprising the vector according to claim 10.

12. Specific binding member or antibody according to claims 1 - 7, a nucleic acid according to claims 8 or 9 or a vector according to claim 10 for use in the treatment of a tumor.

13. Specific binding member or antibody for use according to claim 12 wherein the tumor is a rhabdomyosarcoma.

14. Method for the in vitro labeling of tumor cells expressing a fetal acetylcholine receptor wherein said tumor cell is contacted with a specific binding member or antibody, according to claims 1 - 7, a nucleic acid according to claims 8 or 9 or a vector according to claim 10.


SEQUENCE LISTING

<110>  UNIVERSITEIT MAASTRICHT
       ACADEMISCH ZIEKENHUIS MAASTRICHT

<120>  HUMAN MONOCLONAL ANTIBODY AGAINST THE GAMMA SUBUNIT OF THE
       ACETYLCHOLINE RECEPTOR FOR USE IN THE TREATMENT OF
       RHABDOMYOSARCOMA

<130>  206 EPP0

<160>  39

<170>  PatentIn version 3.5

<210>  1
<211>  297
<212>  DNA
<213>  homo sapiens

<400>  1
gcgtctggat tcaccttctc ggactttggc ctgcactggg tccgccaggc tccagtcagg
60

ggcctgcaat gggtggcatt tatacggaat gatgaatcta gacgatatgg aaaaagtgat
120

caatattatg tcgacgccgt gagggacaga ttcatcatct ccagagacaa ctccgcgaac
180

accttatatc ttcaaatgaa caatgtgaca cttcaagaca cgggtatata ttactgcgcg
240

agagagatga cagcacgtgg caggtcttgg ttcgacccct ggggccaggg aacccag
297


<210>  2
<211>  276
<212>  DNA
<213>  homo sapiens

<400>  2
tccttcctgt ctgcatctgt gggagacaga gtcaccatca cttgccgggc cagtcaaagc
60

atcaacaatt ttttagcctg gtatcagcaa aagccgtcgc agggccttaa ggtcctaatc
120

tatactgcat ccactttgca aagtggagtc ccttcaagat tcagtggcag tggatctggg
180

acagaattca ctctcacaat cagcagcctg caacctgaag attttgcaac ttattactgt
240

caacaaacta atactttccc tctgactttc ggcgga
276

<210>  3
<211>  18
<212>  DNA
<213>  homo sapiens

<400>  3
gactttggcc tgcactgg
18



<210>  4
<211>  69
<212>  DNA
<213>  homo sapiens

<400>  4
tttatacgga atgatgaatc tagacgatat ggaaaaagtg atcaatatta tgtcgacgcc
60

gtgagggac
69



<210>  5
<211>  42
<212>  DNA
<213>  homo sapiens

<400>  5
gcgagagaga tgacagcacg tggcaggtct tggttcgacc cc
42



<210>  6
<211>  33
<212>  DNA
<213>  homo sapiens

<400>  6
cgggccagtc aaagcatcaa caatttttta gcc
33



<210>  7
<211>  33
<212>  DNA
<213>  homo sapiens

<400>  7
actgcatcca ctttgcaaag tggagtccct tca
33



<210>  8
<211>  27
<212>  DNA
<213>  homo sapiens

<400>  8

caacaaacta atactttccc tctgact
27

<210> 9
<211> 99
<212> PRT
<213> homo sapiens

<400> 9

Ala Ser Gly Phe Thr Phe Ser Asp Phe Gly Leu His Trp Val Arg Gln
1               5                   10                  15

Ala Pro Val Arg Gly Leu Gln Trp Val Ala Phe Ile Arg Asn Asp Glu
            20                  25                  30

Ser Arg Arg Tyr Gly Lys Ser Asp Gln Tyr Tyr Val Asp Ala Val Arg
        35                  40                  45

Asp Arg Phe Ile Ile Ser Arg Asp Asn Ser Ala Asn Thr Leu Tyr Leu
    50                  55                  60

Gln Met Asn Asn Val Thr Leu Gln Asp Thr Gly Ile Tyr Tyr Cys Ala
65                  70                  75                  80

Arg Glu Met Thr Ala Arg Gly Arg Ser Trp Phe Asp Pro Trp Gly Gln
                85                  90                  95

Gly Thr Gln

<210> 10
<211> 92
<212> PRT
<213> homo sapiens

<400> 10

Ser Phe Leu Ser Ala Ser Val Gly Asp Arg Val Thr Ile Thr Cys Arg
1               5                   10                  15

Ala Ser Gln Ser Ile Asn Asn Phe Leu Ala Trp Tyr Gln Gln Lys Pro
            20                  25                  30

Ser Gln Gly Leu Lys Val Leu Ile Tyr Thr Ala Ser Thr Leu Gln Ser
        35                  40                  45

Gly Val Pro Ser Arg Phe Ser Gly Ser Gly Ser Gly Thr Glu Phe Thr

50                  55                  60

Leu Thr Ile Ser Ser Leu Gln Pro Glu Asp Phe Ala Thr Tyr Tyr Cys
65                  70                  75                  80

Gln Gln Thr Asn Thr Phe Pro Leu Thr Phe Gly Gly
                85                  90

<210>  11
<211>  6
<212>  PRT
<213>  homo sapiens

<400>  11

Asp Phe Gly Leu His Trp
1               5

<210>  12
<211>  23
<212>  PRT
<213>  homo sapiens

<400>  12

Phe Ile Arg Asn Asp Glu Ser Arg Arg Tyr Gly Lys Ser Asp Gln Tyr
1               5                   10                  15

Tyr Val Asp Ala Val Arg Asp
            20

<210>  13
<211>  14
<212>  PRT
<213>  homo sapiens

<400>  13

Ala Arg Glu Met Thr Ala Arg Gly Arg Ser Trp Phe Asp Pro
1               5                   10

<210>  14
<211>  11
<212>  PRT
<213>  homo sapiens

<400>  14

Arg Ala Ser Gln Ser Ile Asn Asn Phe Leu Ala
1               5                   10

<210>  15
<211>  11
<212>  PRT
<213>  homo sapiens

<400>  15

Thr Ala Ser Thr Leu Gln Ser Gly Val Pro Ser
1               5                   10



<210>  16
<211>  9
<212>  PRT
<213>  homo sapiens

<400>  16

Gln Gln Thr Asn Thr Phe Pro Leu Thr
1               5



<210>  17
<211>  297
<212>  DNA
<213>  homo sapiens

<400>  17
gtttccggat acaccctcac tgaattatcc atgcactggg tgcgacaggc tcctggaaaa
60

gggcttgagt ggatgggaac ttttgatcgt gaagatggtg aaacaatcta cgcacagcag
120

ttccagggca gagtcaccat gaccgaggac acatctacag acacagcctt catggaactg
180

agcagcctga gatctgagga cacggccgtc tattactgtg caacagattc gaccttattg
240

gattcgcact accaatactg tagtggctgt ctttattatt ggggccaggg aaccctg
297



<210>  18
<211>  279
<212>  DNA
<213>  homo sapiens

<400>  18
gccaccctgt ctgtgtctcc aggggagaga gccaccctct cctgcagggc cagtcacagt
60

gttagcagcc acttagcctg gtaccagcag aaacctggcc aggctcccag gctcctcatc
120

tatggtgcat cgaccagggc cgctggtatc ccagccaggt tcagtggcag tgggtctggg
180

acagagttca cactcaccat cagcagcctg cagtctgaag attttgcagt ttattattgt
240

cagcaatata atgactggcc cctgtacact tttggccag
279



<210>  19
<211>  18
<212>  DNA
<213>  homo sapiens

<400>  19
gaattatcca tgcactgg
18



<210>  20
<211>  51
<212>  DNA
<213>  homo sapiens

<400>  20
acttttgatc gtgaagatgg tgaaacaatc tacgcacagc agttccaggg c
51



<210>  21
<211>  42
<212>  DNA
<213>  homo sapiens

<400>  21
gcaacagatt cgaccttatt ggattcgcac taccaatact gt
42



<210>  22
<211>  33
<212>  DNA
<213>  homo sapiens

<400>  22
agggccagtc acagtgttag cagccactta gcc
33



<210>  23
<211>  33
<212>  DNA
<213>  homo sapiens

<400>  23
ggtgcatcga ccagggccgc tggtatccca gcc
33



<210>  24
<211>  27

<212>  DNA
<213>  homo sapiens

<400>  24
cagcaatata atgactggcc cctgtac
27

<210>  25
<211>  99
<212>  PRT
<213>  homo sapiens

<400>  25

Val Ser Gly Tyr Thr Leu Thr Glu Leu Ser Met His Trp Val Arg Gln
1               5                   10                  15

Ala Pro Gly Lys Gly Leu Glu Trp Met Gly Thr Phe Asp Arg Glu Asp
            20                  25                  30

Gly Glu Thr Ile Tyr Ala Gln Gln Phe Gln Gly Arg Val Thr Met Thr
        35                  40                  45

Glu Asp Thr Ser Thr Asp Thr Ala Phe Met Glu Leu Ser Ser Leu Arg
    50                  55                  60

Ser Glu Asp Thr Ala Val Tyr Tyr Cys Ala Thr Asp Ser Thr Leu Leu
65                  70                  75                  80

Asp Ser His Tyr Gln Tyr Cys Ser Gly Cys Leu Tyr Tyr Trp Gly Gln
                85                  90                  95

Gly Thr Leu

<210>  26
<211>  93
<212>  PRT
<213>  homo sapiens

<400>  26

Ala Thr Leu Ser Val Ser Pro Gly Glu Arg Ala Thr Leu Ser Cys Arg
1               5                   10                  15

Ala Ser His Ser Val Ser Ser His Leu Ala Trp Tyr Gln Gln Lys Pro
            20                  25                  30

Gly Gln Ala Pro Arg Leu Leu Ile Tyr Gly Ala Ser Thr Arg Ala Ala

35                  40                  45

Gly Ile Pro Ala Arg Phe Ser Gly Ser Gly Ser Gly Thr Glu Phe Thr
    50                  55                  60

Leu Thr Ile Ser Ser Leu Gln Ser Glu Asp Phe Ala Val Tyr Tyr Cys
65                  70                  75                  80

Gln Gln Tyr Asn Asp Trp Pro Leu Tyr Thr Phe Gly Gln
                85                  90

<210>  27
<211>  6
<212>  PRT
<213>  homo sapiens

<400>  27

Glu Leu Ser Met His Trp
1               5

<210>  28
<211>  17
<212>  PRT
<213>  homo sapiens

<400>  28

Thr Phe Asp Arg Glu Asp Gly Glu Thr Ile Tyr Ala Gln Gln Phe Gln
1               5                   10                  15

Gly

<210>  29
<211>  14
<212>  PRT
<213>  homo sapiens

<400>  29

Ala Thr Asp Ser Thr Leu Leu Asp Ser His Tyr Gln Tyr Cys
1               5                   10

<210>  30
<211>  11
<212>  PRT
<213>  homo sapiens

<400>  30

Arg Ala Ser His Ser Val Ser Ser His Leu Ala
1               5                   10

<210>  31
<211>  11
<212>  PRT
<213>  homo sapiens

<400>  31

Gly Ala Ser Thr Arg Ala Ala Gly Ile Pro Ala
1               5                   10

<210>  32
<211>  9
<212>  PRT
<213>  homo sapiens

<400>  32

Gln Gln Tyr Asn Asp Trp Pro Leu Tyr
1               5

<210>  33
<211>  23
<212>  DNA
<213>  homo sapiens

<400>  33
gacatccaga tgacccagtc tcc
23

<210>  34
<211>  23
<212>  DNA
<213>  homo sapiens

<400>  34
gatgttgtga tgactcagtc tcc
23

<210>  35
<211>  23
<212>  DNA
<213>  homo sapiens

<400>  35
gaaattgtgt tgacgcagtc tcc
23

<210>  36
<211>  23
<212>  DNA

<213>  homo sapiens

<400>  36
gacatcgtga tgacccagtc tcc
23



<210>  37
<211>  23
<212>  DNA
<213>  homo sapiens

<400>  37
gaaacgacac tcacgcagtc tcc
23



<210>  38
<211>  23
<212>  DNA
<213>  homo sapiens

<400>  38
gaaattgtgc tgactcagtc tcc
23



<210>  39
<211>  27
<212>  DNA
<213>  homo sapiens

<400>  39
cttacgtttg atctccagct tggtccc
27

SEQUENCE LISTING

<110>  UNIVERSITEIT MAASTRICHT
       ACADEMISCH ZIEKENHUIS MAASTRICHT

<120>  HUMAN MONOCLONAL ANTIBODY AGAINST THE GAMMA SUBUNIT OF THE
       ACETYLCHOLINE RECEPTOR FOR USE IN THE TREATMENT OF
       RHABDOMYOSARCOMA

<130>  206 EPP0

<160>  39

<170>  PatentIn version 3.5

<210>  1
<211>  297
<212>  DNA
<213>  homo sapiens

<400>  1
gcgtctggat tcaccttctc ggactttggc ctgcactggg tccgccaggc tccagtcagg      60

ggcctgcaat gggtggcatt tatacggaat gatgaatcta gacgatatgg aaaaagtgat     120

caatattatg tcgacgccgt gagggacaga ttcatcatct ccagagacaa ctccgcgaac     180

accttatatc ttcaaatgaa caatgtgaca cttcaagaca cgggtatata ttactgcgcg     240

agagagatga cagcacgtgg caggtcttgg ttcgacccct ggggccaggg aacccag        297


<210>  2
<211>  276
<212>  DNA
<213>  homo sapiens

<400>  2
tccttcctgt ctgcatctgt gggagacaga gtcaccatca cttgccgggc cagtcaaagc      60

atcaacaatt ttttagcctg gtatcagcaa aagccgtcgc agggccttaa ggtcctaatc     120

tatactgcat ccactttgca aagtggagtc ccttcaagat tcagtggcag tggatctggg     180

acagaattca ctctcacaat cagcagcctg caacctgaag attttgcaac ttattactgt     240

caacaaacta atactttccc tctgactttc ggcgga                               276


<210>  3
<211>  18
<212>  DNA
<213>  homo sapiens

<400>  3
gactttggcc tgcactgg                                                    18


<210>  4
<211>  69
<212>  DNA
<213>  homo sapiens

<400>  4
tttatacgga atgatgaatc tagacgatat ggaaaaagtg atcaatatta tgtcgacgcc      60

gtgagggac                                                              69

<210>  5
<211>  42
<212>  DNA
<213>  homo sapiens

<400>  5
gcgagagaga tgacagcacg tggcaggtct tggttcgacc cc                    42


<210>  6
<211>  33
<212>  DNA
<213>  homo sapiens

<400>  6
cgggccagtc aaagcatcaa caatttttta gcc                              33


<210>  7
<211>  33
<212>  DNA
<213>  homo sapiens

<400>  7
actgcatcca ctttgcaaag tggagtccct tca                              33


<210>  8
<211>  27
<212>  DNA
<213>  homo sapiens

<400>  8
caacaaacta atactttccc tctgact                                     27


<210>  9
<211>  99
<212>  PRT
<213>  homo sapiens

<400>  9

Ala Ser Gly Phe Thr Phe Ser Asp Phe Gly Leu His Trp Val Arg Gln
1               5                   10                  15


Ala Pro Val Arg Gly Leu Gln Trp Val Ala Phe Ile Arg Asn Asp Glu
            20                  25                  30


Ser Arg Arg Tyr Gly Lys Ser Asp Gln Tyr Tyr Val Asp Ala Val Arg
        35                  40                  45


Asp Arg Phe Ile Ile Ser Arg Asp Asn Ser Ala Asn Thr Leu Tyr Leu
    50                  55                  60


Gln Met Asn Asn Val Thr Leu Gln Asp Thr Gly Ile Tyr Tyr Cys Ala
65                  70                  75                  80


Arg Glu Met Thr Ala Arg Gly Arg Ser Trp Phe Asp Pro Trp Gly Gln
                85                  90                  95


Gly Thr Gln

<210>  10
<211>  92
<212>  PRT
<213>  homo sapiens

<400>  10

Ser Phe Leu Ser Ala Ser Val Gly Asp Arg Val Thr Ile Thr Cys Arg
1               5                   10                  15

Ala Ser Gln Ser Ile Asn Asn Phe Leu Ala Trp Tyr Gln Gln Lys Pro
            20                  25                  30

Ser Gln Gly Leu Lys Val Leu Ile Tyr Thr Ala Ser Thr Leu Gln Ser
        35                  40                  45

Gly Val Pro Ser Arg Phe Ser Gly Ser Gly Ser Gly Thr Glu Phe Thr
    50                  55                  60

Leu Thr Ile Ser Ser Leu Gln Pro Glu Asp Phe Ala Thr Tyr Tyr Cys
65                  70                  75                  80

Gln Gln Thr Asn Thr Phe Pro Leu Thr Phe Gly Gly
                85                  90


<210>  11
<211>  6
<212>  PRT
<213>  homo sapiens

<400>  11

Asp Phe Gly Leu His Trp
1               5


<210>  12
<211>  23
<212>  PRT
<213>  homo sapiens

<400>  12

Phe Ile Arg Asn Asp Glu Ser Arg Arg Tyr Gly Lys Ser Asp Gln Tyr
1               5                   10                  15

Tyr Val Asp Ala Val Arg Asp
            20


<210>  13
<211>  14
<212>  PRT
<213>  homo sapiens

<400>  13

Ala Arg Glu Met Thr Ala Arg Gly Arg Ser Trp Phe Asp Pro
1               5                   10

<210>  14
<211>  11
<212>  PRT
<213>  homo sapiens

<400>  14

Arg Ala Ser Gln Ser Ile Asn Asn Phe Leu Ala
1               5                   10


<210>  15
<211>  11
<212>  PRT
<213>  homo sapiens

<400>  15

Thr Ala Ser Thr Leu Gln Ser Gly Val Pro Ser
1               5                   10


<210>  16
<211>  9
<212>  PRT
<213>  homo sapiens

<400>  16

Gln Gln Thr Asn Thr Phe Pro Leu Thr
1               5


<210>  17
<211>  297
<212>  DNA
<213>  homo sapiens

<400>  17
gtttccggat acaccctcac tgaattatcc atgcactggg tgcgacaggc tcctggaaaa      60

gggcttgagt ggatgggaac ttttgatcgt gaagatggtg aaacaatcta cgcacagcag     120

ttccagggca gagtcaccat gaccgaggac acatctacag acacagcctt catggaactg     180

agcagcctga gatctgagga cacggccgtc tattactgtg caacagattc gaccttattg     240

gattcgcact accaatactg tagtggctgt ctttattatt ggggccaggg aaccctg        297


<210>  18
<211>  279
<212>  DNA
<213>  homo sapiens

<400>  18
gccaccctgt ctgtgtctcc aggggagaga gccaccctct cctgcagggc cagtcacagt      60

gttagcagcc acttagcctg gtaccagcag aaacctggcc aggctcccag gctcctcatc     120

tatggtgcat cgaccagggc cgctggtatc ccagccaggt tcagtggcag tgggtctggg     180

acagagttca cactcaccat cagcagcctg cagtctgaag attttgcagt ttattattgt     240

cagcaatata atgactggcc cctgtacact tttggccag                            279


<210>  19

<211>  18
<212>  DNA
<213>  homo sapiens

<400>  19
gaattatcca tgcactgg                                                    18


<210>  20
<211>  51
<212>  DNA
<213>  homo sapiens

<400>  20
acttttgatc gtgaagatgg tgaaacaatc tacgcacagc agttccaggg c               51


<210>  21
<211>  42
<212>  DNA
<213>  homo sapiens

<400>  21
gcaacagatt cgaccttatt ggattcgcac taccaatact gt                         42


<210>  22
<211>  33
<212>  DNA
<213>  homo sapiens

<400>  22
agggccagtc acagtgttag cagccactta gcc                                   33


<210>  23
<211>  33
<212>  DNA
<213>  homo sapiens

<400>  23
ggtgcatcga ccagggccgc tggtatccca gcc                                   33


<210>  24
<211>  27
<212>  DNA
<213>  homo sapiens

<400>  24
cagcaatata atgactggcc cctgtac                                          27


<210>  25
<211>  99
<212>  PRT
<213>  homo sapiens

<400>  25

Val Ser Gly Tyr Thr Leu Thr Glu Leu Ser Met His Trp Val Arg Gln
1               5                   10                  15


Ala Pro Gly Lys Gly Leu Glu Trp Met Gly Thr Phe Asp Arg Glu Asp
            20                  25                  30


Gly Glu Thr Ile Tyr Ala Gln Gln Phe Gln Gly Arg Val Thr Met Thr

35                  40                  45

Glu Asp Thr Ser Thr Asp Thr Ala Phe Met Glu Leu Ser Ser Leu Arg
    50                  55                  60

Ser Glu Asp Thr Ala Val Tyr Tyr Cys Ala Thr Asp Ser Thr Leu Leu
65                  70                  75                  80

Asp Ser His Tyr Gln Tyr Cys Ser Gly Cys Leu Tyr Tyr Trp Gly Gln
                85                  90                  95

Gly Thr Leu

<210>  26
<211>  93
<212>  PRT
<213>  homo sapiens

<400>  26

Ala Thr Leu Ser Val Ser Pro Gly Glu Arg Ala Thr Leu Ser Cys Arg
1               5                   10                  15

Ala Ser His Ser Val Ser Ser His Leu Ala Trp Tyr Gln Gln Lys Pro
            20                  25                  30

Gly Gln Ala Pro Arg Leu Leu Ile Tyr Gly Ala Ser Thr Arg Ala Ala
        35                  40                  45

Gly Ile Pro Ala Arg Phe Ser Gly Ser Gly Ser Gly Thr Glu Phe Thr
    50                  55                  60

Leu Thr Ile Ser Ser Leu Gln Ser Glu Asp Phe Ala Val Tyr Tyr Cys
65                  70                  75                  80

Gln Gln Tyr Asn Asp Trp Pro Leu Tyr Thr Phe Gly Gln
                85                  90

<210>  27
<211>  6
<212>  PRT
<213>  homo sapiens

<400>  27

Glu Leu Ser Met His Trp
1               5

<210>  28
<211>  17
<212>  PRT
<213>  homo sapiens

<400>  28

Thr Phe Asp Arg Glu Asp Gly Glu Thr Ile Tyr Ala Gln Gln Phe Gln

1               5                   10                  15

Gly

<210>  29
<211>  14
<212>  PRT
<213>  homo sapiens

<400>  29

Ala Thr Asp Ser Thr Leu Leu Asp Ser His Tyr Gln Tyr Cys
1               5                   10

<210>  30
<211>  11
<212>  PRT
<213>  homo sapiens

<400>  30

Arg Ala Ser His Ser Val Ser Ser His Leu Ala
1               5                   10

<210>  31
<211>  11
<212>  PRT
<213>  homo sapiens

<400>  31

Gly Ala Ser Thr Arg Ala Ala Gly Ile Pro Ala
1               5                   10

<210>  32
<211>  9
<212>  PRT
<213>  homo sapiens

<400>  32

Gln Gln Tyr Asn Asp Trp Pro Leu Tyr
1               5

<210>  33
<211>  23
<212>  DNA
<213>  homo sapiens

<400>  33
gacatccaga tgacccagtc tcc                                              23

<210>  34
<211>  23
<212>  DNA
<213>  homo sapiens

<400>  34
gatgttgtga tgactcagtc tcc                                              23

<210>  35
<211>  23
<212>  DNA
<213>  homo sapiens

<400>  35
gaaattgtgt tgacgcagtc tcc                                               23


<210>  36
<211>  23
<212>  DNA
<213>  homo sapiens

<400>  36
gacatcgtga tgacccagtc tcc                                               23


<210>  37
<211>  23
<212>  DNA
<213>  homo sapiens

<400>  37
gaaacgacac tcacgcagtc tcc                                               23


<210>  38
<211>  23
<212>  DNA
<213>  homo sapiens

<400>  38
gaaattgtgc tgactcagtc tcc                                               23


<210>  39
<211>  27
<212>  DNA
<213>  homo sapiens

<400>  39
cttacgtttg atctccagct tggtccc                                           27